Europäisches Patentamt

European Patent Office

Office européen des brevets

(19)

(11) **EP 1 214 122 B1**

(12) **FASCICULE DE BREVET EUROPEEN**

(45) Date de publication et mention
de la délivrance du brevet:
**24.11.2004 Bulletin 2004/48**

(21) Numéro de dépôt: **00962605.2**

(22) Date de dépôt: **12.09.2000**

(51) Int Cl.⁷: **A61N 7/02**, G01R 33/48

(86) Numéro de dépôt international:
**PCT/FR2000/002506**

(87) Numéro de publication internationale:
**WO 2001/019457 (22.03.2001 Gazette 2001/12)**

(54) **ENSEMBLE DE TRAITEMENT THERMIQUE DE TISSUS BIOLOGIQUES**

ANORDNUNG ZUR THERMISCHEN BEHANDLUNG VON BIOLOGISCHEM GEWEBE

SET FOR HEAT TREATMENT OF BIOLOGICAL TISSUES

(84) Etats contractants désignés:
**AT BE CH CY DE DK ES FI FR GB GR IE IT LI LU
MC NL PT SE**

(30) Priorité: **13.09.1999 FR 9911418**

(43) Date de publication de la demande:
**19.06.2002 Bulletin 2002/25**

(73) Titulaire: **CENTRE NATIONAL DE
LA RECHERCHE SCIENTIFIQUE (CNRS)
75794 Paris Cedex 16 (FR)**

(72) Inventeurs:
• **SALOMIR, Rares-Vasile
F-33800 Bordeaux (FR)**
• **de ZWART, Jacobus, Adrianus
Chevy Chase, Maryland 20815-6539 (US)**
• **VIMEUX, Frédéric
31700 Blaignac (FR)**
• **MOONEN, Christ
F-33170 Gradignan (FR)**

(74) Mandataire: **Texier, Christian et al
Cabinet Régimbeau
20, rue de Chazelles
75847 Paris cedex 17 (FR)**

(56) Documents cités:
EP-A- 0 627 206
US-A- 5 307 812
EP-A- 0 734 742
US-A- 5 443 068

## Description

L'invention concerne le domaine des appareils destinés aux thérapies par hyperthermie locale. Un procédé de mise en oeuvre de tels appareils est également divulgué.

Les thérapies par hyperthermie locale consistent à chauffer, localement, une zone cible d'un tissu biologique. Lorsque ce type de thérapie est utilisé dans le cadre de la thérapie génique, la chaleur peut être, par exemple, utilisée pour son action sur un promoteur thermosensible. On peut également utiliser la chaleur pour nécroser des tissus biologiques et procéder à l'ablation de tumeurs.

Aussi, les thérapies par hyperthermie locale offrent-elles de nombreux avantages. Ces avantages sont aussi bien qualitatifs qu'économiques. Du point de vue qualitatif, elles offrent, par exemple, un fort potentiel pour le contrôle de traitements tels que les thérapies géniques, le dépôt local de médicaments, l'ablation de tumeurs, etc. Du point de vue économique, elles sont compatibles avec un traitement ambulatoire des malades, elles permettent de réduire les durées d'hospitalisation, etc.

Dans les thérapies par hyperthermie, la chaleur peut être, par exemple, apportée par un laser, des micro-ondes ou des ondes radiofréquences, des ultrasons focalisés, etc. D'une manière générale, les thérapies par hyperthermie locale permettent des interventions médicales dont la nature invasive est réduite au minimum. Mais parmi les types d'énergie précités, les ultrasons focalisés sont particulièrement intéressants puisqu'ils permettent de chauffer la zone de focalisation, de manière non invasive, profondément dans un corps biologique, sans chauffer de manière significative les tissus voisins de la zone de focalisation.

Dans tous les cas, la température de la zone cible et de son environnement immédiat, pendant le traitement, doit être contrôlée précisément et de manière continue, alors que l'apport en énergie est localisé et rapide (de l'ordre de quelques secondes). A cette fin, on peut installer des sondes de température dans la zone cible et son environnement immédiat. Mais on peut également utiliser l'Imagerie par Résonance Magnétique (IRM). L'IRM permet, en effet, d'obtenir une cartographie précise des distributions de températures ainsi que des informations anatomiques détaillées. L'IRM permet en outre un contrôle non invasif de la température.

On connaît déjà des dispositifs pour le contrôle de la température, pendant les traitements par des ultrasons focalisés, basés sur la thermométrie par résonance magnétique. De tels dispositifs sont en particulier décrits dans les documents suivants: "Control system for an MRI compatible intracavitary ultrasons array for thermal treatment of prostate disease", Smith NB et al., Proceedings of the annual meeting of the International Society of Magnetic Resonance in Medicine, 1999, p. 672 et "Real time control of focused ultrasound heating based on rapid MR thermometry", Vimeux FC et al., *Invest. Radiol.* 1999, 34, p. 190-193. Dans les dispositifs décrits dans ces documents, le rétrocontrôle de la chaleur apportée par les ultrasons focalisés, grâce aux cartographies obtenues par IRM, est de type PID (acronyme de l'expression anglo-saxonne Proportional Integral and Derivative). De plus, avec ces dispositifs, le contrôle de la chaleur fournie au tissu, repose sur la prise en compte d'une température mesurée dans la zone de focalisation des ultrasons uniquement, ou correspondant à une moyenne obtenue à partir de la distribution spatiale de la température, dans la zone cartographiée.

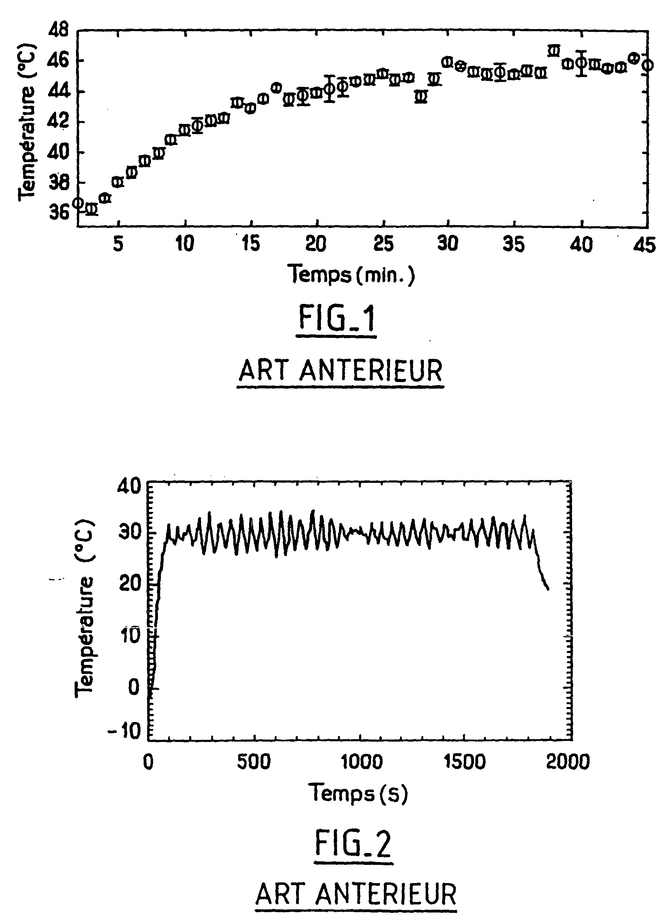

La figure 1 représente l'évolution temporelle de la température moyennée de la zone de focalisation, traitée grâce au dispositif décrit dans le premier de ces documents. Sur cette figure, la température évolue jusqu'à un plateau correspondant à la température que l'on souhaite atteindre dans la zone de focalisation. On peut constater que la température souhaitée dans la zone de focalisation n'est atteinte qu'après une durée de l'ordre de 30 minutes. La figure 2 représente l'évolution temporelle de la température moyennée dans la zone de focalisation, traitée grâce au dispositif décrit dans le deuxième des documents cités ci-dessus. On peut constater que la température souhaitée dans la zone de focalisation est atteinte en moins de 2 minutes. Par contre, on observe des variations de la température souhaitée, de plus ou moins 4° C.

Un but de l'invention est de proposer un ensemble pour le traitement thermique d'une zone cible d'un tissu biologique permettant à la fois d'obtenir rapidement la température souhaitée dans la zone cible et de maintenir et contrôler la température dans cette zone cible avec une précision accrue, par rapport à ce qui était possible avec les techniques de l'art antérieur.

Ce but est atteint, selon l'invention, grâce à un ensemble pour le traitement thermique d'une zone cible d'un tissu biologique, comprenant

- des moyens générateurs d'énergie pour fournir localement de l'énergie dans la zone cible ;
- des moyens pour mesurer et enregistrer la température dans la zone cible;
- une unité de contrôle comprenant des moyens pour déterminer, à partir de la température mesurée dans la zone cible, la quantité d'énergie devant être fournie à la zone cible, et des moyens pour commander aux moyens générateurs de l'énergie, de délivrer cette valeur de la puissance ;

caractérisé par le fait que l'unité de contrôle comprend en outre des moyens de traitement numérique, point par point, de la distribution spatiale de la température dans la zone cible et son environnement, pour calculer des gradients de température.

L'ensemble de traitement thermique conforme à la présente invention, prend en compte la distribution spatiale réelle de la température dans la zone cible, mais aussi dans l'environnement de cette zone. C'est à dire qu'il prend en compte et traite, point par point, cette distribution spatiale. Contrairement aux ensembles de traitement thermique de l'art antérieur, la distribution spatiale de la température est utilisée pour en déduire des gradients de température et non pas de simples moyennes. Ceci permet d'estimer, avec plus de précision, quelle doit être la quantité d'énergie à déposer et donc de parvenir plus rapidement à la température souhaitée et de maintenir la température du tissu biologique avec une plus grande stabilité.

Avantageusement, l'unité de contrôle de l'ensemble de traitement thermique selon l'invention, comprend en outre des moyens pour estimer les pertes locales en énergie thermique, à partir d'une estimation de la conduction thermique et de la distribution spatiale de la température dans la zone cible et son environnement. En effet, l'information fournie par la valeur des gradients de température, ainsi que la prise en compte d'une estimation des pertes locales de chaleur, permettent non seulement de connaître la façon dont le tissu biologique traité a réagi à la chaleur déjà appliquée à celui-ci, mais permettent en outre, grâce à la prévision sur la façon dont le tissu biologique va réagir à la chaleur. Ceci permet également de faire évoluer la température du tissu traité thermiquement, plus rapidement vers la température souhaitée et de maintenir la température du tissu biologique avec une plus grande stabilité.

Avantageusement, les moyens générateurs d'énergie de l'ensemble de traitement thermique selon l'invention, émettent des ultrasons focalisés. En effet, les ultrasons focalisés permettent de fournir de la chaleur, dans une zone très localisée, de manière non invasive, même si cette zone est située profondément dans un corps humain ou animal. En outre la focalisation permet de ne pas chauffer de façon significative, les tissus avoisinant la zone de tissu biologique traitée.

Avantageusement, les moyens pour mesurer et enregistrer la distribution spatiale de la température de l'ensemble de traitement thermique selon l'invention, comprennent un appareil d'imagerie par résonance magnétique. En effet, l'IRM permet une mesure de la température, non invasive, précise, et bien résolue en de nombreux points de la zone cartographiée. Les données recueillies par IRM sont, en outre, aisément traitées numériquement.

Avantageusement, l'ensemble de taitement thermique selon l'invention comprend des moyens d'évaluation de la distribution spatiale, dans la zone cible et son environnement, de l'énergie fournie à la zone cible.

Selon un autre aspect, l'invention est un procédé de régulation d'un ensemble de traitement thermique d'une zone cible d'un tissu biologique, comprenant l'étape consistant à déposer localement de l'énergie dans la zone cible,
   caractérisé par le fait qu'il comprend en outre les étapes consistant à

- évaluer les gradients de température dans la zone cible et son environnement ; et
- en déduire l'énergie à déposer dans la zone cible pour atteindre la température souhaitée.

Avantageusement, ce procédé comprend alors en outre l'étape consistant à estimer les pertes locales en énergie, dans la zone cible et son environnement.

Avantageusement, ce procédé comprend en outre l'étape consistant à évaluer la distribution spatiale, dans la zone cible et son environnement, de l'énergie fournie à la zone cible.

D'autres aspects, buts et avantages de l'invention apparaîtront plus clairement à la lecture de la description détaillée qui suit.

L'invention sera aussi mieux comprise à l'aide des dessins joints sur lesquels:

- la figure 1 représente l'évolution temporelle de la température d'une zone cible, lorsque celle-ci est traitée en utilisant un ensemble de traitement thermique de l'art antérieur ;
- la figure 2 représente l'évolution temporelle de la température d'une zone cible traitée par un autre ensemble de traitement thermique de l'art antérieur ;
- la figure 3 représente schématiquement l'ensemble de traitement thermique conforme à l'invention ;
- la figure 4 est un organigramme du procédé de régulation conforme à la présente invention ;
- la figure 5 représente l'évolution du Laplacien en fonction du temps, au cours du traitement thermique par l'ensemble conforme à la présente invention, correspondant à une expérience *in vitro* décrite ci-dessous ;
- la figure 6 présente les résultats d'une série d'expériences visant à étudier l'influence des erreurs d'estimation sur les pertes d'énergie, représentées par la diffusivité de la chaleur, et le coefficient d'absorption de l'énergie ultrasonore ;
- la figure 7 représente l'évolution temporelle de la température maximum, mesurée au cours du traitement thermique d'un tissu biologique, par l'ensemble conforme à la présente invention, correspondant à la même expérience *in vitro* que celle de la figure 5 ;

- la figure 8 représente l'évolution temporelle de l'amplitude du signal émis par le générateur de l'ensemble conforme à la présente invention, au cours du traitement thermique d'un tissu biologique, correspondant à même expérience *in vitro* que celle des figures 5 et 7 ;
- la figure 9 représente l'évolution temporelle de la température maximum, au cours du traitement thermique, d'un tissu biologique, par l'ensemble conforme à la présente invention, correspondant à une autre expérience *in vitro,* avec trois paliers de température, et décrite ci-dessous ; et
- la figure 10 représente l'évolution temporelle de la température maximum, mesurée au cours du traitement thermique par l'ensemble conforme à la présente invention, correspondant à une expérience *in vivo* décrite ci-dessous.

Un des modes de réalisation de l'invention, est décrit ci-dessous de manière détaillée. A titre d'exemple, ce mode de réalisation de l'invention correspond à un ensemble de traitement par hyperthermie locale par ultrasons focalisés, contrôlés par IRM.

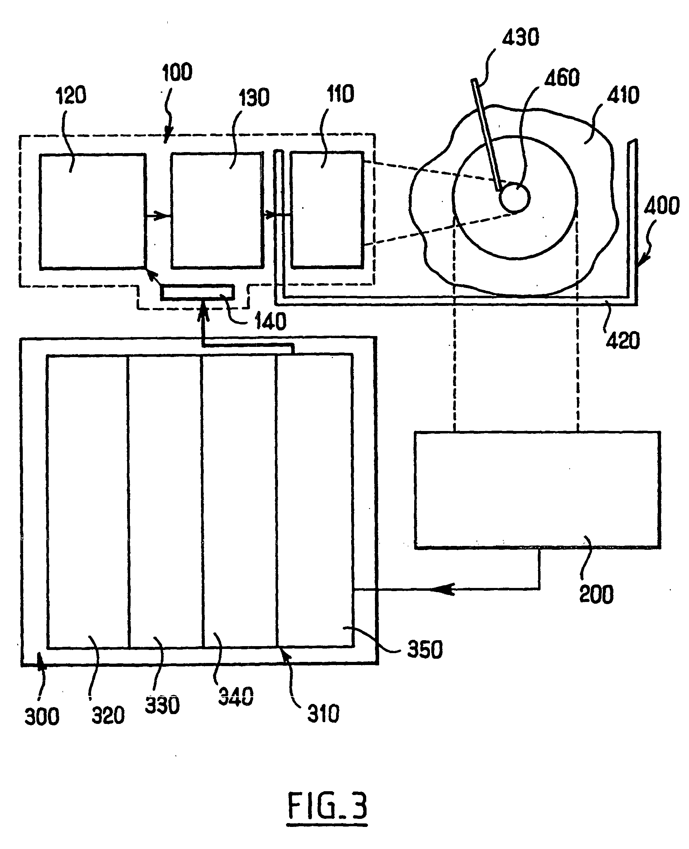

Comme représenté sur la figure 3, un tel ensemble comprend :

- des moyens générateurs d'énergie 100 ;
- des moyens de cartographie 200 ;
- une unité de contrôle 300 ; et
- un porte-échantillon 400 pour le tissu biologique 410 à traiter.

Dans le mode de réalisation de l'invention décrit ici, les moyens générateurs d'énergie 100 sont composés d'un transducteur 110, d'un générateur de signal sinusoïdal 120, d'un amplificateur 130 et d'un convertisseur 140, reliant le générateur de signal sinusoïdal 120, à l'unité de contrôle 300.

Le transducteur 110 opère à 1,45 MHz. Un tel type de transducteur 110 est, par exemple, commercialisé par la société Speciality Engineering Associates® (Soquel, Californie). Son diamètre et sa distance focale sont de 38 mm et 25 mm respectivement.

Le générateur de signal sinusoïdal 120 est, par exemple, du type FG110, commercialisé par la société Yokogawa® (Tokyo, Japon).

L'amplificateur 130 est, par exemple, du type KMP 170F, commercialisé par la société Kalmus® (Bothell, Washington). Cet amplifiacteur 130 a un gain en puissance de 58 dB.

Le convertisseur 140 est, par exemple un convertisseur IEEE488 série, commercialisé par la société I. O. Tech.® (Cleveland, Ohio).

Les moyens de cartographie 200, permettent de mesurer et enregistrer la distribution spatiale de la température. Ils comprennent, par exemple, un appareil IRM de type Bruker Biospec commercialisé par la société Bruker® (Ettlingen, Allemagne). Cet appareil utilise un aimant de 4,7 T qui est équipé d'un insert de 120 mm de diamètre, qui génère des gradients du champ magnétique (la valeur maximum du gradient est de 0,193 T/m).

L'unité de contrôle 300 comprend en particulier une station de travail 310 de type Alpha PW 500a MHz, commercialisée par la société Digital®.

L'unité de contrôle 300 comporte également des moyens d'évaluation et de traitement numérique de la distribution spatiale de la température 320, des moyens de détermination de la valeur de la puissance 330 devant être fournie à la zone cible, des moyens d'estimation des pertes locales de l'énergie thermique 340 et des moyens de commande 350 des moyens générateurs d'énergie 100. Les moyens de commande 350 indiquent aux moyens générateurs d'énergie 100 de délivrer la valeur de la puissance fournie par les moyens de détermination de la valeur de la puissance 330.

Le porte-échantillon 400 comprend un support 420 de rat, en plexiglas®. Ce support 420 contient le transducteur 110 et une bobine de surface (non représentée sur la figure 3). Un tel porte échantillon 400 a déjà été décrit dans les documents « Fast lipid supressed MR temperature mapping with echo-shifted gradient echo imaging and spectral-spatial excitation », de Zwart JA et al., 1999, *Magn. Res.Med*., 42, p.53-59 ; et « On the feasibility of MRI-guided focused ultrasound for local induction of gene expression », Madio DP et al., 1998, *J. Magn. Res. Imaging. I*, 8, p. 101-104. Le support 420 est placé dans un tube de plexiglas® qui est partiellement rempli d'eau. Le transducteur 110 est positionné de manière à ce que le point de focalisation 460 des ultrasons soit situé approximativement à 10mm en profondeur, dans le tissu biologique 410. Pendant les mesures *in vitro,* une sonde 430 de température est insérée dans le tissu biologique 410 constitué d'un morceau de viande fraîche, de manière à avoir une référence pour la température. Cette sonde 430 est par exemple un thermocouple du type Digi-Sence DualLog, commercialisé par la société Cole-Parmer Instrument Co.® (Vemon Hill, Illinois).

La préparation des échantillons pour les expériences *in vitro* et *in vivo* est réalisées de la manière suivante. On prend des rats mâles de la race Wistar de 325 à 500 g. Ceux-ci sont anesthésiés en associant 1% en volume d'halothane avec un mélange constitué de 7 volumes d'oxyde nitreux ($N_2O$) pour 3 volumes d'oxygène, selon un protocole approuvé. Afin d'améliorer la pénétration du faisceau d'ultrasons dans le tissu biologique 410, la cuisse du rat (qui se trouve entre transducteur et point focal) est épilée, en utilisant un produit prévu à cet effet et connu de l'homme du métier. Pendant les mesures *in vivo*, la température endorectale des rats est enregistrée. La température corporelle des rats est maintenue à 35°C, par immersion du corps des rats dans un bain dont la température est régulée à cette fin. Après le traitement thermique les rats sont sacrifiés.

Les moyens de cartographie 200 sont utilisés en mettant en oeuvre une séquence Echo gradient, avec les paramètres suivants : TR = 50 ms, TE = 15 ms, la taille de la matrice = 64x63, trois lignes de l'espace des k par TR, FOV = 64 mm x 63 mm, où TR est le temps de répétition, TE est le temps d'écho et FOV est le champ de vue (FOV est l'acronyme de l'expression anglosaxone "Field of View"). Les données sont obtenues par IRM, à partir d'une coupe de 2 mm d'épaisseur, perpendiculaire au transducteur à ultrasons et comprenant le point focal des ultrasons focalisés. La résolution temporelle des cartographies obtenues par IRM est de 1,05 s. La résolution spatiale des cartographies obtenues par IRM est de 1x1x2 mm³. Les cartographies de la température mesurée par résonance magnétique sont obtenues à partir des mesures du déplacement de la fréquence du proton de l'eau. Le choix de la résonance du proton de l'eau, pour faire ces mesures, repose sur le fait que la relation entre la fréquence de résonance du proton de l'eau et la température est, en première approximation, indépendante de la composition du tissu biologique 410. Le déplacement de la fréquence du proton de l'eau en fonction de la température est en outre linéaire et cette linéarité n'est pas affectée par les modifications du tissu biologique 410 induites par la chaleur (Ishihara Y et al., Magn. Res. Med., 1995, 34, p. 814-823 ; Peters RD et al., *Magn. Res. Med.,* 1998, 40, p. 454-459). La dépendance en température de la fréquence de résonance du proton est de 0,0094 ppm-K$^{-1}$ (« Fast magnetic-resonance temperature imaging » ;de Zwart JA et al, 1996, *J. Mag. Res. B,* 112, p. 86-90 ; « Fast lipid supressed MR temperature mapping with echo-shifted gradient echo imaging and spectral-spatial excitation », de Zwart JA et al., 1999, *Magn. Res. Med.,* 42, p.53-59).

Les signaux de résonance magnétique provenant des lipides constituent une source significative d'erreurs dans les cartographies de température calculées, puisque les fréquences de résonance des protons des lipides ne dépendent pas de la température. On supprime donc les signaux de résonance magnétique provenant des lipides, en utilisant une excitation sélective de l'eau, de la manière décrite dans le document « Fast lipid supressed MR temperature mapping with echo-shifted gradient echo imaging and spectral-spatial excitation », de Zwart JA et al., 1999, *Magn. Res.Med*., 42, p.53-59.

L'utilisation des moyens d'évaluation et de traitement numérique de la distribution spatiale de la température 320, a également déjà été décrit dans le document « Fast lipid supressed MR temperature mapping with echo-shifted gradient echo imaging and spectral-spatial excitation », de Zwart JA et al., 1999, *Magn. Res. Med*., 42, p.53-59.

Un exemple de mise en oeuvre du procédé de régulation de l'ensemble de traitement thermique, conforme à la présente invention est décrit de manière détaillée ci-dessous.

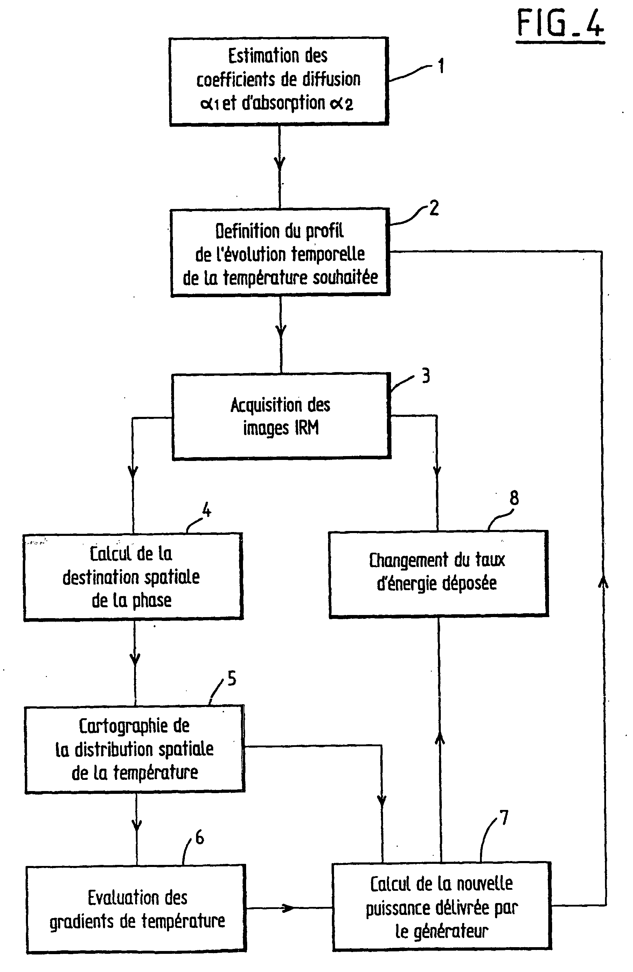

Sur la figure 4, est représenté schématiquement, un organigramme de ce mode particulier de mise en oeuvre du procédé selon l'invention.

Conformément à cet exemple de mise en oeuvre, le procédé selon l'invention comporte :

- une étape 1 d'estimation du coefficient de diffusion de la chaleur ($\alpha_1$) et du coefficient d'absorption des ultrasons focalisés ($\alpha_2$) dans le tissus biologique 410 ;
- une étape 2 de définition par l'utilisateur, d'un profil de l'évolution temporelle de la température souhaitée ;
- une étape 3 d'acquisition d'une image IRM ;
- une étape 4 de calcul de la distribution spatiale de la phase au point focal 460 et dans son environnement ;
- une étape 5 d'établissement de la distribution spatiale de la température au point focal 460 et son environnement ;
- une étape 6 d'évaluation des gradients de température du point focal 460 et dans son environnement ;
- une étape 7 de détermination de la nouvelle puissance devant être délivrée par le générateur 120 ; et
- une étape 8 de changement du taux d'énergie déposée par le générateur 120.

Les étapes 3 à 8 sont reprises en boucle, afin d'atteindre et de parcourir le profil de l'évolution temporelle de la température souhaitée, défini à l'étape 2.

Pour un élément donné du transducteur 110, la puissance électrique *P(t)* transmise à un échantillon est déterminée par les moyens de détermination de la valeur de la puissance 320. Sa valeur peut donc être modifiée en direct par l'unité de contrôle 300. Elle est obtenue sur la base de l'équation :

$$P(t) = \frac{1}{\alpha_2(T_{\max})}\left[\frac{d\Theta(t)}{dt} - \alpha_1(T_{\max}) \cdot \nabla^2 T_{\max}(t) + a \cdot \left[\Theta(t) - T_{\max}(t)\right] + \frac{a^2}{4} \cdot \Delta(t)\right]$$

Cette équation est établie en se fondant sur les considérations suivantes.

Le point focal est défini par $\vec{r}$ = (0,0,0). L'évolution temporelle et la température maximum au point focal correspond alors à $T_{max}(t) = T(0,0,0,t)$. Notons par $\Theta(t)$ le profil prédéterminé de l'évolution temporelle souhaitée de la température maximum $T_{max}(t)$. Comme indiqué ci-dessus, ce profil est défini avant le début de chaque expérience. Par exemple, ce profil $\Theta(t)$ comporte une montée de 10° C, pendant 100 s. Cette étape de montée suit l'évolution d'une moitié de la période de la fonction cosinus. Elle est suivie par une période où la température est constante (10° C au-dessus de la valeur de départ), pendant 250 s.

La température maximum $T_{max}(t) = T(0,0,0,t)$ peut être contrôlée seulement au point focal. En effet, la géométrie du transducteur 110 et la distribution spatiale de l'indice de réfraction dans le tissu biologique 410 détermine le champ acoustique. En conséquence, l'évolution de la température, ailleurs que dans la zone de focalisation, fait intervenir des fonctions dépendantes de la coordonnée d'espace $\vec{r}$ et de la température $T$. Le champ de la puissance acoustique $\rho(\vec{r})$, le tenseur de la diffusivité de la chaleur $\hat{\alpha}_1(\vec{r},T)$ dans le tissu biologique 410 et le coefficient d'absorption des ultrasons focalisés $\alpha_2(\vec{r},T)$, sont alors liés par la relation :

$$\frac{\partial T(\vec{r},t)}{\partial t} = \vec{\nabla}[\hat{\alpha}_1(\vec{r},T)\cdot\vec{\nabla}T(\vec{r},t)] + \alpha_2(\vec{r},T)\cdot\rho(\vec{r})\cdot P(t). \qquad [1a]$$

Lorsque la diffusivité est isotrope et varie lentement spacialement, l'équation 1a se simplifie pour donner l'équation 1b, où $\alpha_1(\vec{r},T) = \frac{1}{3}Tr[\hat{\alpha}_1(\vec{r},T)]$ est un champ scalaire et $\nabla^2$ est l'opérateur Laplacien défini par $\frac{\partial^2}{\partial x^2} + \frac{\partial^2}{\partial y^2} + \frac{\partial^2}{\partial z^2}$ :

$$\frac{\partial T(\vec{r},t)}{\partial t} = \alpha_1(\vec{r},T)\cdot\nabla^2 T(\vec{r},t) + \alpha_2(\vec{r},T)\cdot\rho(\vec{r})\cdot P(t) \qquad [1b]$$

Il faut noter que les fonctions $\alpha_1(\vec{r},T)$ et $\alpha_2(\vec{r},T)$ ne sont pas précisément connues au début du chauffage.

Le champ de la puissance acoustique d'un élément sphérique de transducteur à ultrasons focalisés, correspond approximativement à une distribution gaussienne autour du point focal 460, avec un rayon d'atténuation à 6dB noté $R_0$. Le temps de diffusion spécifique $\tau$ est défini par

$$\tau = \frac{R_o^2}{2\alpha_1}.$$

Pour une longueur d'onde ultrasonore de 1 mm, l'ordre de grandeur de $\tau$ est de l'ordre de 10 s.

On se fixe les objectifs suivants :

1) la température du point focal 460 doit atteindre la température souhaitée, aussi vite que possible (c'est-à-dire dans un temps du même ordre de grandeur que $\tau$), ceci sans oscillation, ni dépassement de la valeur souhaitée ;
2) une fois cette valeur souhaitée atteinte, la température doit rester constante pendant une période prédéfinie par l'utilisateur.

L'intégrale du profil $\Theta(t)$, l'intégrale de l'évolution temporelle de la température maximum observée expérimentalement $T_{max}(t)$ et la différence entre ces deux intégrales, sont respectivement :

$$\Omega(t) = \int_0^t \Theta(t')dt' \qquad [2a]$$

$$\Psi(t) = \int_0^t T_{max}(t')dt' \qquad [2b]$$

$$\Delta(t) = \Omega(t) - \Psi(t) = \int_0^t [\Theta(t') - T_{max}(t')]dt' \qquad [2c]$$

En utilisant ces expressions, l'équation 1b, donnant l'évolution de la température au point focal, peut être exprimée en fonction de $\Delta(t)$:

$$\frac{d^2\Delta(t)}{dt^2} = \frac{d\Theta(t)}{dt} - \alpha_1(T_{max})\cdot\triangledown^2 T_{max}(t) - \alpha_2(T_{max})\cdot P(t) \qquad [3]$$

ou $\vec{r}$ = (0,0,0) est omis et $\rho(0,0,0)=1$.

Dans l'équation 3, le paramètre que l'on souhaite contrôler directement est la puissance des ultrasons focalisés $P(t)$. Notons qu'une équation différentielle d'ordre 2 et linéaire en $\Delta(t)$, peut être avantageusement utilisée par l'unité de contrôle 300, de manière similaire à un système de contrôle PID. La raison en est que la solution pour $\Delta(t)$ d'une telle équation tend asymptotiquement vers zéro, et qu'il en est de même pour sa dérivée première. Si la dérivée première de $\Delta(t)$ est égale à zéro, $T_{max}(t)$ présente un recouvrement avec le profil prédéterminé de l'évolution temporelle de la température $\Theta(t)$. Ceci constitue l'idée fondamentale du procédé de contrôle mis en oeuvre par l'unité de contrôle 300. Ainsi, nous pouvons réécrire l'équation 3 sous forme d'une équation différentielle linéaire de second ordre en $\Delta(t)$ de type :

$$\frac{d^2\Delta}{dt^2} + a\cdot\frac{d\Delta}{dt} + \frac{a^2}{4}\cdot\Delta = 0 \qquad [4]$$

De manière à obtenir l'expression voulue de l'équation 4 à partir de l'équation 3, $P(t)$ est réécrit avec l'expression suivante :

$$P(t) = \frac{1}{\alpha_2(T_{max})}\left[\frac{d\Theta(t)}{dt} - \alpha_1(T_{max})\cdot\nabla^2 T_{max}(t) + a\cdot\left[\Theta(t) - T_{max}(t)\right] + \frac{a^2}{4}\cdot\Delta(t)\right] \qquad [5]$$

L'équation 5 correspond à l'équation centrale utilisée pour calculer en direct le niveau de puissance des ultrasons focalisés. A partir de la solution de l'équation différentielle du second ordre, correspondant à l'équation 4, on peut voir que le paramètre $a$ est relié au temps de réponse caractéristique $t_r$ de la boucle de régulation, par l'expression $a=2/t_r$. On suppose que dans les équations 4 et 5, toutes les fonctions utilisées pour calculer la puissance $P(t)$ sont précisément connues. On peut également vérifier, comme le montre l'équation 6 ci-dessous, que la température observée expérimentalement $T(t)$, tend asymptotiquement vers le profil $\Theta(t)$ :

$$\Theta(t) - T(t) = \frac{d\Delta}{dt}(t) = \left[\Theta(0) - T(0)\right]\cdot\left(1 - \frac{a}{2}\cdot t\right)\cdot\exp\left(-\frac{a}{2}\cdot t\right) \qquad [6]$$

Comme déjà noté plus haut, dans une expérience réelle, les coefficients d'absorption des ultrasons $\alpha_2$ et le paramètre de diffusion de la chaleur $\alpha_1$, ainsi que leur dépendance en température, sont inconnus. Ces paramètres $\alpha_1$ et $\alpha_2$ dépendent de la composition du tissu biologique 410, de processus physiologiques, telle que la perfusion, et de changements irréversibles intervenant pendant la procédure de chauffage, par exemple dans les procédures d'ablation. Ainsi, un système de régulation doit il être tolérant vis à vis des erreurs sur les paramètres $\alpha_1$ et $\alpha_2$.

Seul le profil $\Theta(t)$ et sa dérivée sont précisément connues.

Aussi, lorsque la puissance ultrasonore est calculée en direct à partir de l'équation 5, deux difficultés apparaissent :

1) $T_{max}(t)$ et $\triangledown^2 T_{max}(t)$ telles qu'elles sont obtenues à partir de la cartographie de la température issue de l'IRM, sont affectées par le bruit.
2) les valeurs de $\alpha_1$ et $\alpha_2$ et leur dépendance en température ne sont pas précisément connues, de même leur sensibilité à la nécrose par chauffage (par exemple dans les procédures d'ablation) et les paramètres physiologiques telle que la perfusion, ne sont pas connus avec précision.

Toute erreur pouvant affecter $\alpha_1$ et $\alpha_2$ peut être traitée comme une erreur de paramètre dans une boucle de contrôle, selon un modèle linéaire. Ainsi, des estimations des valeurs initiales de $\alpha_1$ et $\alpha_2$ seront choisies, puis utilisées pendant la procédure de chauffage, pour le calcul de la puissance ultrasonore, conformément à l'équation 5.

Une analyse théorique de l'effet de l'erreur sur les paramètres $\alpha_1$ et $\alpha_2$ dans l'équation 5 met en évidence les effets suivants :

1) Une estimation erronée du paramètre $\alpha_2$ diminue l'efficacité de la boucle de régulation ; il peut en effet s'ensuivre une possibilité de dépassement de la température souhaitée ou une valeur de la température déterminée trop faible ; ceci a alors pour conséquence une augmentation du temps de convergence. Quoiqu'il en soit, même dans ces conditions, la température expérimentale tend toujours asymptotiquement vers le profil prédéterminé de l'évolution temporelle de la température.

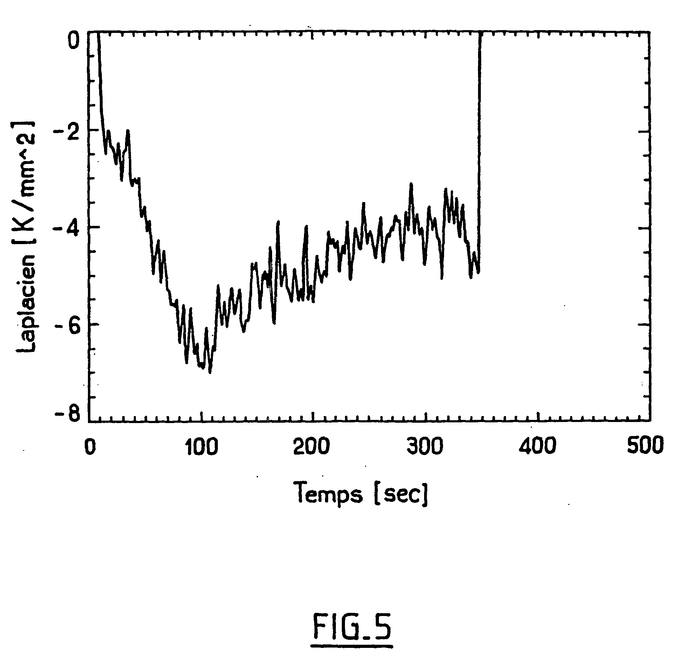

2) Une estimation erronée du paramètre $\alpha_1$ conduit à un décalage constant, dans la zone où le profil $\Theta(t)$ est plat, des valeurs de la température, entre les valeurs de la température mesurées expérimentalement et le profil $\Theta(t)$. Ce décalage est proportionnel à la dérivée première par rapport au temps, du Laplacien, multipliée par l'erreur absolue sur $a_1$, fois $a^{-2}$. Afin d'estimer cet effet, la dérivée du Laplacien a été déterminée en utilisant une régression linéaire de la courbe représentée sur la figure 5, entre 150 et 250 s. Sa valeur est de l'ordre de 0.01 K.mm$^{-2}$.s$^{-1}$, ce qui conduit à un décalage en température approximativement égal à 0.1°C. Ainsi, l'erreur sur la température réelle ne devrait pas être directement observée du fait de la limitation de la précision des mesures thermométriques par résonance magnétique, à cause du bruit.

L'influence de l'erreur sur les valeurs $\alpha_1$ et $\alpha_2$ sur l'efficacité du contrôle de la température par l'ensemble de traitement thermique conforme à l'invention est étudiée expérimentalement, sur un morceau de viande fraîche, ci-dessous.

Selon l'étape 1, les paramètres $\alpha_1$ et $\alpha_2$ doivent être estimés. Ceci est réalisé, dans un premier temps à partir d'une expérience préliminaire avec une puissance d'ultrasons focalisés constante. Le paramètre $\alpha_1$ est calculé à partir de la dérivée par rapport au temps, de la température au point focal, divisée par la moyenne (sur cinq images IRM) du Laplacien. Il est calculé immédiatement après l'extinction de l'émission des ultrasons focalisés et est exprimé en mm$^2$/s. Le paramètre $\alpha_2$ (la vitesse de dépôt de l'énergie au point focal prenant en compte la distribution spatiale de la puissance des ultrasons focalisés) est calculé à partir de la dérivée, au temps initial, de la température du point focal, par rapport à la puissance des ultrasons focalisés (lorsque les ultrasons focalisés sont émis et que la diffusion est négligeable, voir l'équation 1). Il est exprimé en K.s$^{-1}$.(mV)$^{-2}$. La précision estimée pour $\alpha_1$ et $\alpha_2$ est meilleure que 10%, comme on peut le déduire d'expériences répétées. Les valeurs numériques obtenues de cette façon peuvent être directement utilisées dans l'équation 5 pour calculer, à chaque fois qu'une nouvelle cartographie de la température est disponible, la valeur réelle de la puissance devant être fournie par le générateur 120.

Selon l'étape 2, un profil de l'évolution temporelle souhaitée de la température au point focal est défini avant le début de chaque expérience. Ce profil comporte une partie initiale croissante, correspondant à une moitié de la période de la fonction cosinus, suivi d'une partie constante en température. La dérivée première de la courbe correspondant à ce profil, est continue et peut être calculée numériquement par ordinateur.

L'étape 3 est mise en oeuvre grâce aux moyens de cartographie 200.

Les étapes 4 à 6 sont mises en oeuvre grâce aux moyens d'évaluation de traitement numérique de la distribution spatiale de la température 320. Les phases sont calculées à partir des signaux IRM, obtenus grâce aux moyens de cartographie 200. Les changements de fréquence de résonance de l'eau sont calculées à partir de ces phases. Les changements de température sont calculés à partir de ces changements de fréquence.

La température maximum $T_{max}(t)$ et l'intégrale $\Delta(t)$ sont directement déduites des cartographie obtenus par IRM, respectivement au cours des étapes 5 et 6. L'intégrale $\Delta(t)$ (Equation 2c) est calculée numériquement à l'aide de la station de travail 310.

Au cours de l'étape 6, les pertes locales de l'énergie thermique sont réévaluées à l'aide des moyens d'estimation des pertes locales de l'énergie thermique 340.

Selon l'étape 7 du procédé conforme à la présente invention, l'opérateur Laplacien $\triangledown^2 T(\vec{r},t)$ est appliqué au traitement des cartographies dé la température obtenues par IRM. La valeur de ce Laplacien au point focal est calculé, en utilisant la méthode des éléments fins, en combinaison avec un filtre temporel de réduction du bruit. Ce filtre utilise un rapport de pondération binomial égal à 1 :4 :6 :4 :1, sur cinq images. Cette étape 7 est mise en oeuvre avec les moyens de détermination de la valeur de la puissance 330.

Finalement, la puissance est calculée grâce à l'équation 5 et la valeur calculée est envoyée au convertisseur 140 (étape 8). L'étape 8 est mise en oeuvre avec les moyens de commande 350 des moyens générateurs d'énergie 100.

La durée totale de calcul pour traiter chaque cartographie de distribution spatiale de la température, c'est à dire chaque cycle de l'ensemble des étapes 3 à 8 décrites ci-dessus, est inférieure à 250ms.

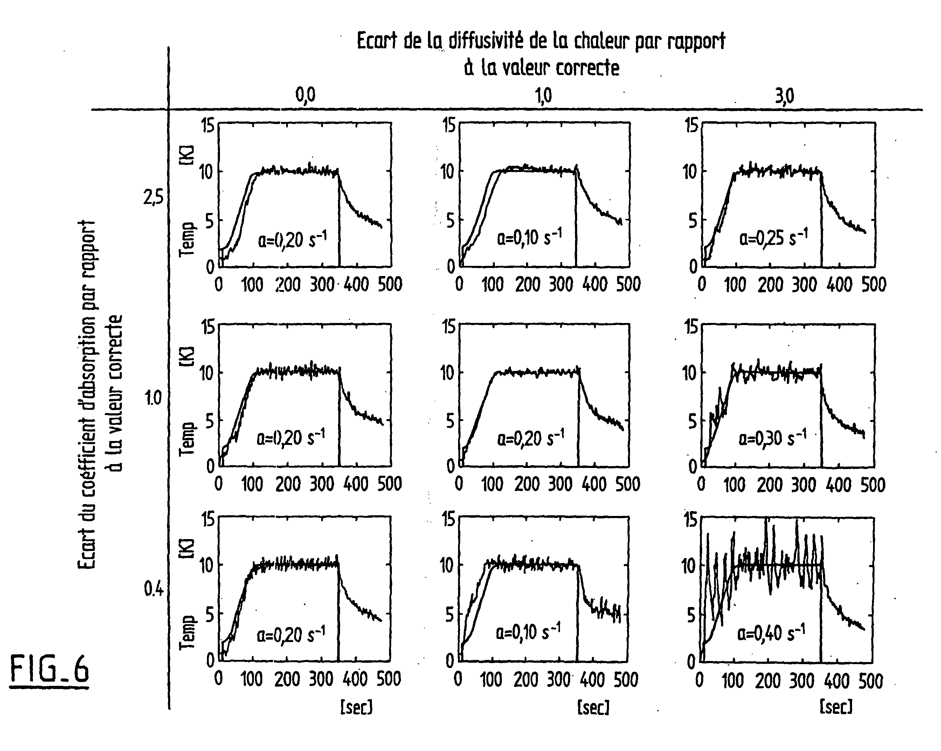

Afin d'analyser la tolérance vis-à-vis des erreurs dans les estimations initiales de $\alpha_1$ et $\alpha_2$, plusieurs procédures de chauffage ont été mises en oeuvre avec les paramètres $\alpha_1$ et $\alpha_2$ variant sur une large gamme. Cette gamme va de 0 à 300 % d'une valeur prédéterminée, pour $\alpha_1$, et de 40 à 250 %, pour $\alpha_2$. Cette valeur prédéterminée est celle obtenue à partir des mesures préliminaires exposées ci-dessus. Des temps d'attente de 30 minutes ont été introduits entre les expériences successives dans le but d'atteindre les lignes de base en température, spatialement uniformes dans l'échantillon et identiques pour chaque expérience. Neuf résultats représentatifs sont reportés sur la figure 6. Ces résultats montrent que le système a une large tolérance vis-à-vis des erreurs sur les estimations des valeurs initiales de $\alpha_1$ et $\alpha_2$. Quoi qu'il en soit, on peut noter que la boucle de contrôle devient instable seulement lorsque $\alpha_1$ est fortement surestimé. Cette instabilité est exacerbée lorsque $\alpha_1$ est fortement surestimé et $\alpha_2$ est sous-estimée. Cet effet peut être attribué au bruit expérimental des mesures de la température par IRM. Lorsqu'un calcul affecté par le bruit, conduit à une surestimation de la valeur du Laplacien (les dérivées secondes étant sensibles au bruit), la puissance des ultrasons focalisés appliqués augmente dans une proportion égale à $\alpha_1.\varepsilon/\alpha_2$, où $\varepsilon$ est la surestimation du Laplacien. Une augmentation de la puissance des ultrasons focalisés conduit à une forte augmentation du Laplacien dans le tissu biologique 410 et de ce fait, à une nouvelle augmentation de la puissance des ultrasons focalisés. Cette réaction positive cessera après un temps approximativement égal à $2/a$, grâce à une réaction négative dans la boucle de contrôle. Ceci explique la périodicité des instabilités dans ce cas extrême.

La force de la réaction négative sur la boucle de régulation correspond au paramètre $a$. En effet, comme nous l'avons vu plus haut le paramètre $a$, est égal à deux fois l'inverse du temps de réponse caractéristique $t_r$ ($a=2/ t_r$). La valeur de ce paramètre est indiquée pour chaque exemple de la figure 6. En général, les valeurs de $a$ de 0,1 s$^{-1}$ à 0,2 s$^{-1}$ sont suffisantes pour atteindre des temps de montée en température similaires à ceux du profil $\Theta(t)$, même lorsque des valeurs fortement erronées de $\alpha_1$ et $\alpha_2$ sont utilisées. C'est seulement dans le cas extrême où $\alpha_1$ est fortement sous-estimé et $\alpha_2$ est surestimé (voir figure 6, en bas à droite), que $a$ doit être augmentée jusqu'à la valeur de 0,40 s$^{-1}$ pour obtenir un recouvrement avec le profil prédéterminé de l'évolution temporelle de la température. La valeur de $a$ optimale, expérimentalement trouvée (dans tous les cas, sauf les cas d'erreurs extrêmes sur $\alpha_1$ et $\alpha_2$), est de 0,2 s$^{-1}$, le temps de réponse de la boucle de régulation correspondante étant de 10 s. Lorsque la force de la réaction négative est augmentée, une correction plus rapide des erreurs sur les paramètres initiaux est obtenue, mais l'amplitude de la puissance des ultrasons focalisés et des fluctuations en température autour de la valeur prédéterminée est aussi augmentée.

La mise en oeuvre et les performances de l'ensemble de traitement selon l'invention décrit ci-dessus, sont illustrées ci-dessous à l'aide de deux exemples.

Exemple 1 : Utilisation de l'ensemble de traitement thermique, conforme à la présente invention, dans le cadre de mesures *in vitro*

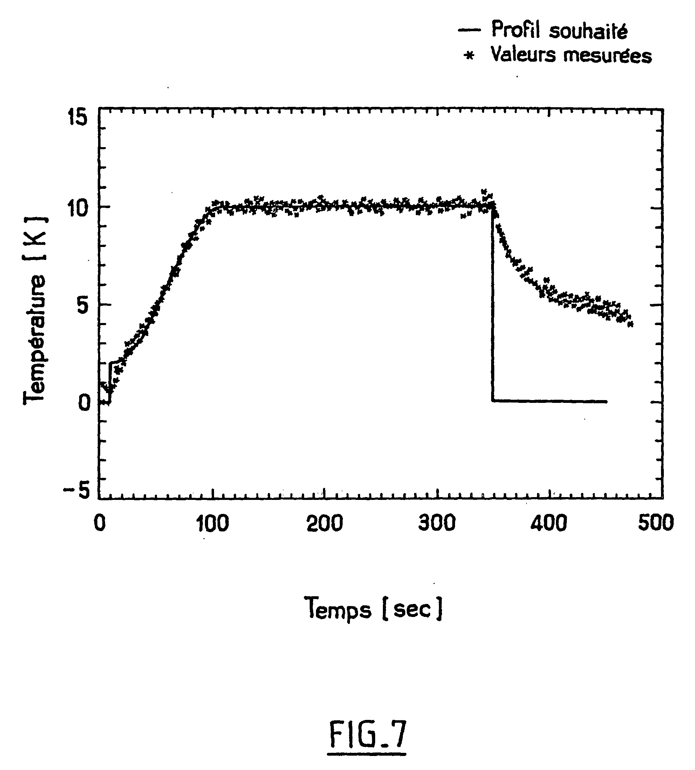

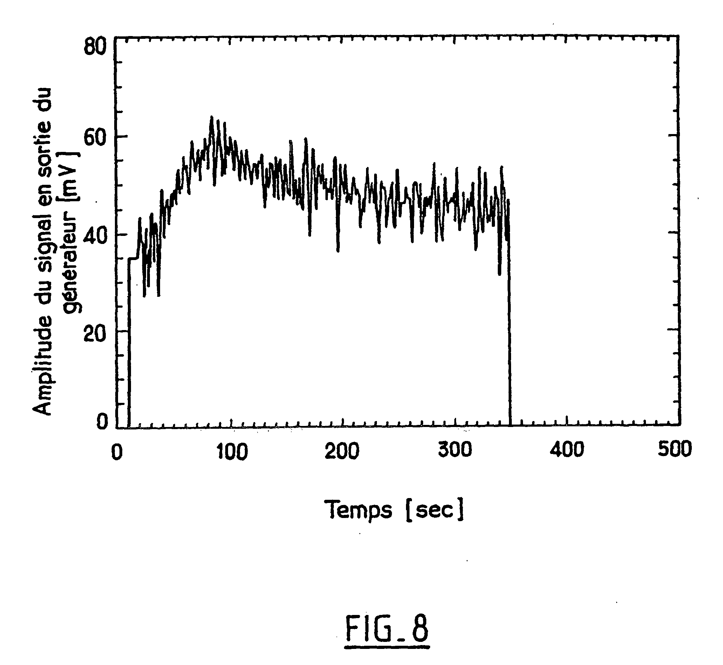

Selon cet exemple, un protocole de montée en température de 10°C est mis en oeuvre sur un échantillon de viande fraîche. La température initiale est égale à 15°C. Avec ce protocole, aucune modification irréversible du tissu biologique 410, résultant de ce profil d'évolution temporelle de la température, n'est attendue. La figure 7 représente l'évolution de la température maximum en fonction du temps. Dans la partie plate de cette courbe, l'élévation moyenne de la température est de 9.97°C, avec un écart type égal à 0.19°C. Cet écart type doit être comparé à celui, égal à 0.18°C, qui est obtenu par les mesures de température effectuées sans chauffage par ultrasons focalisés (c'est à dire ce qui correspond à la ligne de base du bruit dans les mesures de température). Sur la figure 5, est reporté le Laplacien calculé en direct. L'atténuation observée sur la partie constante en température correspond à la diminution des gradients en température autour du point focal. L'amplitude de la puissance appliquée en direct est reportée, quant à elle, sur la figure 8. A cause du bruit de mesure, la valeur calculée du Laplacien et l'amplitude de la puissance fournie par le générateur, présentent une fluctuation de l'ordre de 10%, approximativement. Ceci n'a qu'un faible effet sur la température résultante, car la fréquence de fluctuation (c'est à dire l'inverse de la résolution temporelle de la cartographie par résonance magnétique) est beaucoup plus grande que l'inverse du temps de réponse ($\tau$) spécifique au chauffage du tissu biologique 410.

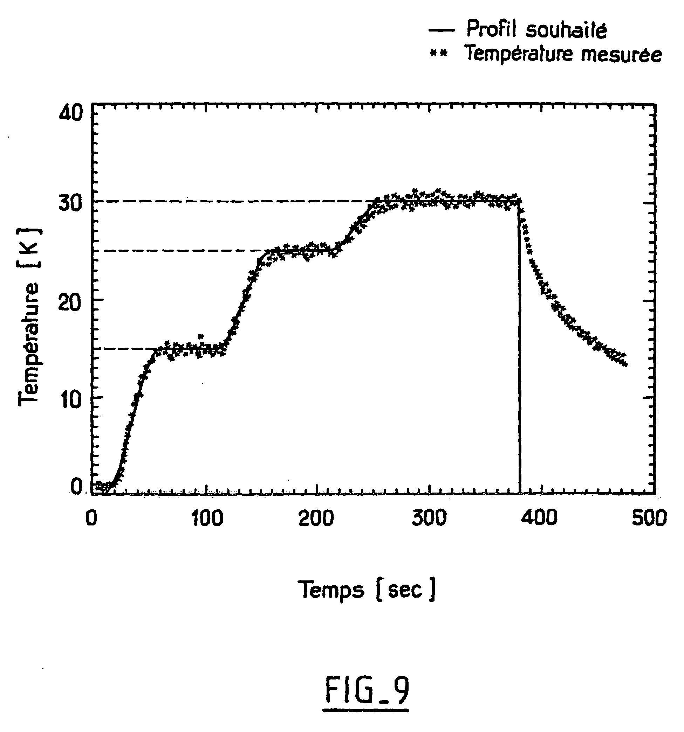

La figure 9 montre la stabilité en température obtenue avec un profil à trois paliers (15, 25, 30 °C). L'écart type est égal à 0.35°C, 0.36°C et 0.40°C, respectivement pour des montées en température à 15°C, 25°C et 30°C. Les résultats représentés sur la figure 9 confirment la grande stabilité en température, sur une large gamme de montée en température, du système de régulation de l'ensemble de traitement thermique conforme à la présente invention.

Exemple 2 : Utilisation de l'ensemble de traitement thermique, conforme à la présente invention, dans le cadre de mesures *in vivo*.

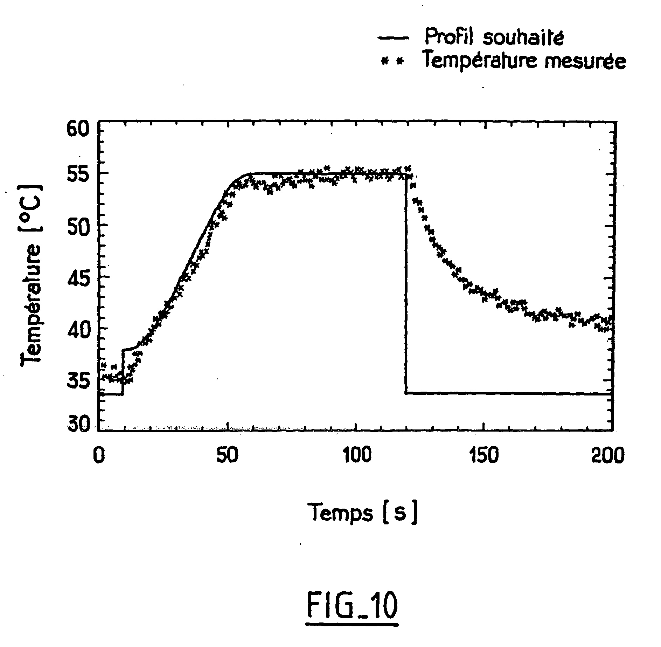

En adoptant une procédure analogue à celle mise en oeuvre dans le cas de l'exemple 1, des expériences ont été menées *in-vivo*, sur une cuisse de rat. Les résultats correspondants sont montrés sur la figure 10. La résolution temporelle est de 0.5s. La température moyenne, entre 90 et 120 s après le début de l'expérience, est de 54.9°C (la valeur du profil à atteindre est égale à 55°C) avec un écart type de 0,33°C. Les figures 7, 9 et 10 montrent que l'on peut contrôler la température avec une précision proche de celle donnée par les mesures de température effectuées *in vitro* ou *in vivo*.

Il a été décrit ci-dessus un mode de réalisation de l'invention correspondant à un ensemble de traitement par hyperthermie locale par ultrasons focalisés, contrôlés par IRM, mais l'invention couvre une gamme plus large d'ensembles de traitement thermique. Aussi, on comprendra que l'invention peut être généralisée aux cas où la chaleur est, par exemple, apportée par un laser, des micro-ondes ou des ondes radiofréquence, des ultrasons focalisés, etc. On comprendra également que d'autres moyens de mesure de la température, peuvent être utilisés dans l'ensemble de traitement thermique conforme à la présente invention, à la place de l'IRM.

De même l'évaluation et la traitement numérique de la distribution spatiale de la température ont été décrits ci-dessus comme étant effectués à l'aide du Laplacien. D'autres moyens d'effectuer cette évaluation peuvent être utilisés sans sortir du cadre de l'invention ;

**Revendications**

1. Ensemble pour le traitement thermique d'une zone cible d'un tissu biologique (410), comprenant

   - des moyens générateurs d'énergie (100) pour fournir localement de l'énergie dans la zone cible ;
   - des moyens (200) pour mesurer et enregistrer la température dans la zone cible;
   - une unité de contrôle (300) comprenant des moyens (330) pour déterminer, à partir de la température mesurée dans la zone cible, la quantité d'énergie devant être fournie à la zone cible, et des moyens pour commander (350) aux moyens générateurs de l'énergie (100), de délivrer cette valeur de la puissance ;

   **caractérisé par le fait que** l'unité de contrôle (300) comprend en outre des moyens de traitement numérique (320), point par point, de la distribution spatiale de la température dans la zone cible et son environnement, pour calculer des gradients de température.

2. Ensemble de traitement thermique selon la revendication 1, **caractérisé par le fait que** l'unité de contrôle (300) comprend en outre des moyens (340) pour estimer les pertes locales en énergie thermique, à partir d'une estimation de la conduction thermique et de la distribution spatiale de la température dans la zone cible et son environnement.

3. Ensemble de traitement thermique selon l'une des revendications précédentes, **caractérisé par le fait que** les moyens générateurs d'énergie (100) émettent des ultrasons focalisés.

4. Ensemble de traitement thermique selon l'une des revendications précédentes **caractérisé par le fait que** les moyens (200) pour mesurer et enregistrer la distribution spatiale de la température comprennent un appareil d'imagerie par résonance magnétique.

5. Ensemble de traitement thermique selon l'une des revendications précédentes, **caractérisé en ce qu'**il comprend des moyens d'évaluation de la distribution spatiale, dans la zone cible et son environnement, de l'énergie fournie à la zone cible.

**Claims**

1. Equipment for the heat treatment of a target zone of biological tissue (410), comprising:

   - energy generating means (100) for suppling energy locally in the target zone;
   - means (200) for measuring and recording the temperature in the target zone;
   - a control unit (300) comprising means (330) for determining, from the temperature measured in the target zone, the amount of energy having to be supplied to the target zone, and means for controlling (350) the energy generating means (100) to deliver this power value;

   **characterized in that** the control unit (300) furthermore comprises means (320) for numerically processing, point by point, the spatial temperature distribution in the target zone and its surroundings, in order to calculate temperature gradients.

**2.** Heat treatment equipment according to Claim 1, **characterized in that** the control unit (300) furthermore comprises means (340) for estimating the local heat energy losses, from an estimate of the heat conduction and of the spatial temperature distribution in the target zone and its surroundings.

**3.** Heat treatment equipment according to either of the preceding claims, **characterized in that** the energy generating means (100) emit focused ultrasound.

**4.** Heat treatment equipment according to one of the preceding claims, **characterized in that** the means (200) for measuring and recording the spatial temperature distribution comprise a magnetic resonance imaging apparatus.

**5.** Heat treatment equipment according to one of the preceding claims, **characterized in that** it comprises means for evaluating the spatial distribution, in the target zone and its surroundings, of the energy supplied to the target zone.

**Patentansprüche**

**1.** Vorrichtung zur thermischen Behandlung eines Zielbereichs eines biologischen Gewebes (410), die aufweist

- Energieerzeugungsmittel (100), um in den Zielbereich lokal Energie zuzuführen;
- Mittel (200) zur Messung und Aufzeichnung der Temperatur im Zielbereich;
- eine Steuereinheit (300), die Mittel (330) umfaßt, um, ausgehend von der im Zielbereich gemessenen Temperatur, die Energiemenge zu ermitteln, die dem Zielbereich zugeführt werden soll, sowie Mittel (350), um die Energieerzeugungsmittel (100) so anzusteuern, daß sie diesen Leistungswert bereitstellen;

**dadurch gekennzeichnet, daß** die Steuereinheit (300) außerdem Mittel (320) zur punktweisen numerischen Bearbeitung der räumlichen Temperaturverteilung im Zielbereich und dessen Umgebung umfaßt, um Temperaturgradienten zu berechnen.

**2.** Anordnung zur thermischen Behandlung nach Anspruch 1, **dadurch gekennzeichnet, daß** die Steuereinheit (300) außerdem Mittel (340) umfaßt, um, ausgehend von einer Abschätzung der Wärmeleitung und der räumlichen Temperaturverteilung im Zielbereich und dessen Umgebung, die lokalen Verluste an thermischer Energie abzuschätzen.

**3.** Anordnung zur thermischen Behandlung nach einem der voranstehenden Ansprüche, **dadurch gekennzeichnet, daß** die Energieerzeugungsmittel (100) fokussierten Ultraschall aussenden.

**4.** Anordnung zur thermischen Behandlung nach einem der voranstehenden Ansprüche, **dadurch gekennzeichnet, daß** die Mittel (200) zur Messung und Aufzeichnung der räumlichen Temperaturverteilung ein Gerät zur Bildgebung mittels magnetischer Resonanz umfassen.

**5.** Anordnung zur thermischen Behandlung nach einem der voranstehenden Ansprüche, **dadurch gekennzeichnet, daß** sie Mittel zur Abschätzung, im Zielbereich und dessen Umgebung, der räumlichen Verteilung der dem Zielbereich zugeführten Energie umfaßt.

**FIG. 1**

**ART ANTERIEUR**

**FIG. 2**

**ART ANTERIEUR**

FIG.3

FIG.4

FIG.5

FIG_6

FIG.7

FIG.8

FIG_9

FIG_10